# EUROPÄISCHE PATENTSCHRIFT

(11) **EP 4 101 427 B1**
(45) Veröffentlichungstag und Bekanntmachung des Hinweises auf die Patenterteilung: **03.09.2025**
(21) Anmeldenummer: 22183475.7
(22) Anmeldetag: 04.09.2019
(51) Int. Cl.: A61F 13/01, A61F 13/05, A61F 13/00

(54) **WUNDREINIGUNGSEINRICHTUNG**
WOUND CLEANSING DEVICE
DISPOSITIF DE NETTOYAGE DE PLAIES

(30) Priorität: 04.09.2018 DE 102018121501; 08.01.2019 DE 202019100062 U
(43) Veröffentlichungstag der Anmeldung: 14.12.2022
(62) Teilanmeldung aus: 19765978.2
(73) Patentinhaber: Lohmann & Rauscher GmbH, 1140 Wien (AT)
(72) Erfinder: HENTRICH, Axel, 1060 Wien (AT); SCHMALENBACH, Carina, 2753 Markt Piesting (AT); HÄUSLER, Cornelia, 12030 Wien (AT)
(74) Vertreter: Herrmann, Daniel

(56) Entgegenhaltungen:
- EP-A1- 2 777 662
- WO-A1-2010/085831
- DE-A1- 102006 049 108
- DE-A1- 102012 100 842
- US-A- 3 561 441
- US-A1- 2003 079 324
- US-A1- 2015 157 508

## Beschreibung

Die Erfindung betrifft eine Wundreinigungseinrichtung mit einer Anzahl von strangförmigen Reinigungselementen aufweisenden Wundreinigungsschicht. Derartige Wundreinigungseinrichtungen sind beispielsweise in der EP 2 365 794 angegeben. Bei der in dieser Schrift beschriebenen Wundreinigungseinrichtung sind die strangförmigen Reinigungselemente in Form von Fäden aus synthetischen Fasern ausgeführt, wobei die Fäden auch in Form von Monofilamentfasern vorliegen können. In der EP 2777662 A1 sind Wundreinigungseinrichtungen mit schlaufenförmigen Reinigungselementen beschrieben. In der US 3.561,441 ist ein Wundverband beschrieben, bei dem der Wundkontakt aus nicht haftenden Schlaufen hergestellt wird und kürzere Schlaufen zum Absorbieren von Flüssigkeit vorgesehen sind. Eine abrasive und absorbierende Wundreinigungseinrichtung ist in der US 2003/007924 A1 angegeben. Eine weitere Wundreinigungseinrichtung ist aus WO2010/085831 bekannt.

Mit den bekannten Wundreinigungseinrichtungen kann das sogenannte Debridement besonders schonend durchgeführt werden. Beim Debridement handelt es sich um den Vorgang der Wundbettpräparation, bei dem vom Körper selbst gebildete Substanzen, also Humanmaterial, wie zum Beispiel überschießende Flüssigkeiten, Fibrinbeläge, abgestorbenes Gewebe der Oberhaut, wie etwa überschießendes Hornmaterial oder tote Hornzellen und/oder Beläge aus abgestorbenem Gewebe (Nekrosen) entfernt werden. Die bekannte Wundreinigungseinrichtung ist als Wundreinigungstuch ausgeführt, wobei die als Fäden verwirklichten Wundreinigungselemente von einer Trägerschicht abstehen. Sie bilden einen Flor, bei dem die auch als Florhöhe bezeichnete wirksame Länge der Fäden zwischen der Trägerschicht und dem der Trägerschicht abgewandten Ende der Fäden zwischen 3 und 30 mm beträgt und die Fäden zwischen 0,5 und 20 dtex aufweisen.

Wenngleich die bekannten Wundreinigungseinrichtungen bei der Behandlung vieler Wunden, insbesondere stark exsudierender Wunden, mit guten Erfolg eingesetzt werden können, hat es sich gezeigt, dass die Wundreinigung unter Einsatz der bekannten Wundreinigungseinrichtungen in anderen Fällen Probleme bereitet.

Angesichts dieser Probleme im Stand der Technik liegt der Erfindung die Aufgabe zugrunde, eine Wundreinigungseinrichtung für erweiterte Einsatzbereiche bereitzustellen.

Erfindungsgemäß wird diese Aufgabe durch die im Patentanspruch 1 angegebene Weiterbildung der bekannten Wundreinigungseinrichtungen gelöst.

Es hat sich gezeigt, dass die nur mangelhafte Wundreinigung unter Verwendung der bekannten Wundreinigungseinrichtungen auf den Einsatz besonders weicher und langer Fasern zurückzuführen ist, die zwar eine schonende Wundbettpräparation gewährleisten, aber in vielen Fällen nicht dazu geeignet sind, hartnäckig anhaftende Substanzen, wie sie etwa bei Brandwunden, Nekrosen oder einigen Fibrinbelägen vorkommen, aus der Wunde abzulösen und/oder aufzunehmen.

Im Rahmen der Erfindung wurde erkannt, dass insbesondere bei der Behandlung der gerade genannten Brandwunden, Nekrosen und hartnäckigen Fibrinbelägen ein ausgewogener Kompromiss zwischen zufriedenstellender Reinigung einerseits und noch akzeptabler mechanischer Belastung der Wunde im Verlauf der Reinigung gefunden werden kann, wenn der Reinigungsquotient im Bereich zwischen 1 und 100 N/mm, vorzugsweise zwischen 10 und 100 N/mm eingestellt wird.

Erfindungsgemäß sind die Reinigungselemente in Form von von einer Trägerschicht ausgehenden Schlaufen ausgeführt. Die wirksame Länge der Reinigungselemente ist als die halbe Länge der Schlaufe definiert, gemessen zwischen den beiden Punkten, an denen sie aus der Trägerschicht austritt. Die so definierte wirksame Länge der Schlaufen kann auch als Florhöhe eines aus diesen Schlaufen gebildeten Flors bezeichnet werden.

Bei schlaufenförmigen Reinigungselementen hat es sich als zweckmäßig erwiesen, wenn zumindest einige dieser Reinigungselemente als ggf. verdrillte Bündel von zwei, drei oder mehr Monofilamenten ausgeführt sind. Dabei können die Monofilamente dieser Reinigungselemente einen Reinigungsquotienten von weniger als 0,05 N/mm aufweisen, sofern der Reinigungsquotient der durch die Monofilamente gebildeten Bündel größer als 1 N/mm ist. Im Rahmen der Erfindung bezeichnet der Ausdruck verdrillt eine Struktur, bei der die einzelnen Monofilamente eine gemeinsame Wendelachse umlaufen.

Eine Beschädigung der einzelnen Reinigungselemente noch ausreichend verhindernde Biegsamkeit der Reinigungselemente kann erreicht werden, wenn die Fasern einen Durchmesser von 500 µm oder weniger, insbesondere 150 µm oder weniger aufweisen. Andererseits kann ein Herauslösen einzelner Fasern ohne übermäßige Verdichtung des Vlieses noch zufriedenstellend verhindert werden, wenn der Durchmesser der Fasern 10 µm oder mehr, vorzugsweise mehr als 20 µm, insbesondere mehr als 30 µm oder mehr, besonders bevorzugt 45 µm oder mehr aufweist. Dabei wird eine übermäßige Verdichtung des Vlieses auch unter dem Gesichtspunkt als problematisch angesehen, dass noch genügend Raum für die aus der Wunde herausgelösten Substanzen in der Reinigungseinrichtung zur Verfügung stehen muss. Ähnlich wie in der EP 2 365 794 erläutert, können diese Substanzen auch bei Einsatz erfindungsgemäßer Reinigungseinrichtungen durch elektrostatische Anziehung in der Reinigungseinrichtung festgehalten werden.

Wenn die Reinigungselemente in Form eines ggf. verdrillten Bündels von zwei, drei oder mehr Monofilamenten ausgeführt sind, können die einzelnen Monofilamente des Bündels auch einen Durchmesser von weniger als 10 µm aufweisen. Aber auch in diesem Fall hat es sich als günstig erwiesen, wenn die Dicke der das Bündel bildenden Monofilamente 5 µm oder mehr beträgt, um dem Herauslösen einzelner Fasern aus dem Bündel entgegenzuwirken.

Zur Bereitstellung einer ausreichenden Aufnahmekapazität für aus der Wunde herausgelöste Verunreinigungen hat es sich im Rahmen der Erfindung als günstig erwiesen, wenn die wirksame Länge der Fasern vorzugsweise 3 mm oder mehr, insbesondere 6 mm oder mehr beträgt. Dabei wird eine Verklebung der einzelnen Reinigungselemente vor Ausnutzung der maximalen Aufnahmekapazität für herausgelöste Verunreinigungen verhindert, wenn die wirksame Länge der Stapelfasern 11 mm oder weniger beträgt.

Erfindungsgemäß ist der Elastizitätsmodul der Chemiefasern 50.000 N/mm² oder weniger, besonders bevorzugt 5000 N/mm² oder weniger und 500 N/mm² oder mehr. Bei Einstellung eines größeren Elastizitätsmoduls muss die Faserlänge ebenfalls entsprechend vergrößert werden und/oder die Querschnittsfläche entsprechend reduziert werden, was zu den oben bereits angesprochenen Problemen führen kann. Die Untergrenze für den Elastizitätsmodul ergibt sich entsprechend.

Erfindungsgemäß bestehen mindestens einige Reinigungselemente zumindest teilweise, vorzugsweise vollständig, aus Polyester. Zur Förderung der Reinigungswirkung ist im Rahmen der Erfindung auch daran gedacht, auf die Oberfläche mindestens eines Reinigungselements ein Schleifmittel aufzubringen. Ein im Rahmen der Erfindung einsetzbares Aramid weist einen Elastizitätsmodul von etwa 100.000 N/mm² auf. Im Rahmen der Erfindung einsetzbares Polyethylen kann einen Elastizitätsmodul zwischen 95.000 und 135.000 N/mm² aufweisen. Zur Herstellung erfindungsgemäßer Wundreinigungseinrichtungen einsetzbare Cellulose kann einen Elastizitätsmodul zwischen 3000 N/mm² (Viskose) und 100.000 N/mm² (Flachs) aufweisen. Stapelfasern aus Polypropylen können einen Elastizitätsmodul von 5000 N/mm² aufweisen. Polyamidfasern können einen Elastizitätsmodul zwischen 250 N/mm² und 3500 N/mm² aufweisen.

Das Schleifmittel kann im Rahmen der Erfindung Korund, Zirkon bzw. Aluminiumoxid, Siliziumcarbid, Bornitrid, Borcarbid, Keramik, Chromoxid, Flint, Quartz, Schmirgel, Granat, Bornitride, insbesondere kubisches Bornitrid und/oder Diamant aufweisen. Dabei kann das Schleifmittel eine Körnung im Bereich zwischen 16 und 1200 Mesh (nach DIN 69176), insbesondere zwischen 150 und 800 Mesh aufweisen.

Erfindungsgemäß weist die Wundreinigungseinrichtung mindestens eine zum Ablösen von Substanzen aus einer Wunde und zum Festhalten der abgelösten Substanzen ausgelegte, eine Anzahl von Wundreinigungselementen aufweisende Wundreinigungsschicht, auf, wobei diese Wundreinigungsschicht zwei, drei oder mehr Wundreinigungsbereiche mit sich voneinander unterscheidenden Wundreinigungseigenschaften umfasst. Die Wundreinigungsbereiche liegen erfindungsgemäß streifenförmig nebeneinander.

Dabei geht die Erfindung auf die Erkenntnis zurück, dass die im Stand der Technik beobachteten Probleme bei der Wundreinigung auch darauf zurückzuführen sind, dass die Anforderungen der Wundreinigung durch geometrisch voneinander getrennte Bereiche besonders gut erfüllt werden können, wenn jeder einzelne Bereich für einen Einzelvorgang der Wundreinigung optimiert ist. Das ist im Besonderen bei der Behandlung von Brandwunden Nekrosen und/oder hartnäckigen Fibrinbelägen von Bedeutung.

Es hat sich gezeigt, dass die geometrische Trennung einzelner Wundreinigungsbereiche mit jeweils optimierten Wundreinigungseigenschaften insgesamt bessere Wundreinigungsergebnisse erbringt, als die Optimierung des Wundreinigungsbereichs insgesamt, wie sie im Stand der Technik angeregt wird, indem spezielle Faserstrukturen vorgeschlagen werden, mit der unterschiedliche Anforderungen mit einem einzigen Reinigungsbereich erfüllt werden können.

Erfindungsgemäß ist einer der Wundreinigungsbereiche ein zum Absorbieren von Wundflüssigkeit, insbesondere seröser Wundflüssigkeit, ausgelegter Absorptionsbereich.Dabei beträgt die wirksame Faserlänge des Absorptionsbereichs vorzugsweise 15 mm oder weniger, insbesondere 10 mm oder weniger, weil bei Einsatz längerer Fasern auch bei der Absorption von serösen Substanzen mit geringer Viskosität keine Verbesserungen mehr erzielt werden. Die aus der Wunde abgelösten Substanzen können zwischen den einzelnen Fasern festgehalten werden. So kann eine erneute Verschmutzung der Wunde durch zuvor abgelöste Substanzen verhindert werden.

Erfindungsgemäß ist einer der Wundreinigungsbereiche ein zum Ablösen von Fibrinbelägen, abgestorbenem Gewebe, Hornmaterial oder dergleichen ausgelegter Abrasionsbereich. Dabei ist mindestens ein Abrasionsbereich zwischen zwei Absorptionsbereichen angeordnet. Der Abrasionsbereich erfindungsgemäßer Wundreinigungseinrichtungen kann Chemiefasern, insbesondere Monofilamente und/oder Multifilamente, mit einer wirksamen Faserlänge von 5 mm oder weniger, vorzugsweise 3 mm oder weniger aufweisen und/oder die wirksame Faserlänge in einem zwischen zwei Absorptionsbereichen angeordneten Abrasionsbereich kann 90 % oder weniger, insbesondere 50 % oder weniger, besonders bevorzugt 30 % oder weniger der wirksamen Faserlänge des Absorptionsbereichs betragen. Dabei beträgt die wirksame Länge der Fasern im Abrasionsbereich vorzugsweise 0,5 mm oder mehr, insbesondere 1,5 mm oder mehr und/oder 5 % oder mehr, insbesondere 10 % oder mehr der wirksamen Faserlänge in benachbarten Absorptionsbereichen.

Überraschenderweise hat es sich gezeigt, dass die kürzeren Fasern des Abrasionsbereichs bedingt durch ihre größere Steifigkeit nicht nur das Ablösen von Substanzen aus der Wunde begünstigen, sondern auch unter besonderen Bedingungen das Festhalten dieser Substanzen verbessern. Dieses überraschende Ergebnis geht darauf zurück, dass insbesondere bei Ablösen von viskosen Exsudaten/Fibrinbelägen Verklebungen im Bereich der Faserspitzen auftreten, welche die Ausnutzung des gesamten zwischen den Fasern des Absorptionsbereichs zur Verfügung stehenden Volumens unmöglich machen. Wenn kürzere Fasern zum Einsatz kommen, welche das Ablösen der Substanzen aus der Wunde begünstigen, wird ein geringerer Grad der Verklebung im Bereich der Faserspitzen beobachtet, weil die Spitzen wegen der geringeren Auslenkung in einem geringeren Umfang zum dauerhaften Verkleben neigen, so dass insgesamt eine größere Absorptionskapazität zwischen den Fasern zur Verfügung gestellt werden kann, auch wenn das verfügbare Volumen insgesamt geringer ist als das zwischen den längeren Fasern verfügbare Absorptionsvolumen. Ferner kann dadurch eine Höhlenbildung im Produkt in Form von ungenutzten Faserbereichen unter den Verklebungen an der Produktoberfläche reduziert werden.

Darüber hinaus können bei der Anordnung von Abrasionsbereichen zwischen Absorptionsbereichen mit größeren Faserlängen abgelöste Substanzen auch im Bereich des Übergangs zwischen Abrasionsbereich und Absorptionsbereich seitlich in den Absorptionsbereich eindringen.

Wundreinigungseinrichtungen, die ausschließlich aus langen Fasern bestehen, werden bei Anfeuchten durch die aufgenommene Flüssigkeit schwer und damit für die Wundreinigung unhandlich. Durch den erfindungsgemäßen Einsatz von Absorptionsbereichen mit langen Fasern und Abrasionsbereichen mit kurzen Fasern wird das Gewicht reduziert und die Dosierung auf der Wunde kann optimiert werden, da man den Wundgrund besser "durchfühlen" und die Wundgrundebenen durch mechanischen Druckangleich an den jeweiligen Bereich gleichmäßig reinigen kann. Wie bereits erwähnt, kann die Kapazität der Wundreinigungseinrichtung besser ausgenutzt werden, weil die höheren Faserlängen auch seitlich (am Übergang zu den niedrigeren Faserbereichen) vom Exsudat/Fibrin erreicht werden können. Auf der anderen Seite reduzieren die längeren Fasern die Härte bzw. Steifigkeit des Produkts, was wiederum zu einer geringeren mechanischen Beanspruchung der Wunde und Wundumgebung beiträgt.

Darüber hinaus können sogenannte Mischwunden (in Wunden sind in der Regel mehrere Viskositäten anzutreffen) effektiv behandelt werden, denn, wie vorstehend bereits erläutert, hat jede einzelne Faserlänge der Reinigungsschicht einen anderen optimalen Funktionsbereich hinsichtlich Effektivität und mechanischer Kapazität, die kurze Faser bei härteren/fibrinartigen Belägen und/oder Flüssigkeiten mit hoher Viskosität bzw. sehr zähflüssigen Substanzen, die lange Faser bei serösem Exsudat (niedrige Viskositäten). Somit können mehrere Funktionsbereiche in einem Produkt vereint werden. Auch ist zu erwarten, dass harte Brocken in der abwechselnden Produktfaserstruktur gut gehalten werden können. Bei Wundreinigungsschichten aus Fasern gleicher Länge neigen feste Brocken dazu, "abzuperlen".

Bei einer anderen Ausführungsform der Erfindung kann die Trägerschicht profiliert sein und die der Trägerschicht abgewandten Begrenzungsflächen der Absorptions- und Abrasionsbereiche etwa in einer gemeinsamen Ebene angeordnet sein. Bei dieser Ausführungsform der Erfindung ist es von besonderem Vorteil, dass sich die längeren Fasern der Absorptionsschicht kaum über die kürzeren Fasern der Abrasionsschicht legen können, weil sie durch die Profilierung der Trägerschicht festgehalten werden. So können Abrasions- und Absorptionseigenschaften auch bei dem während der Wundreinigung auftretenden Druck auf die Wundreinigungseinrichtung beibehalten werden. Derartige Wundreinigungseinrichtungen sind mit besonderem Vorteil einsetzbar, wenn härtere Substanzen oder Beläge, insbesondere Fibrinbeläge und/oder Nekrosen aus einer ansonsten serös exsudierenden Wunde abgelöst werden müssen. Zusätzlich oder alternativ zur Profilierung der Trägerschicht kann die Trägerschicht auch durchgehende Schnittlinien aufweisen, so dass die beidseits der Schnittlinien angeordneten Bereiche der Wundreinigungsschicht unabhängig voneinander an das Profil der zu reinigenden Fläche, wie etwa Wundfläche oder Wundumgebungsfläche, angepasst werden können. In diesem Fall können beidseits der Schnittlinie Fasern gleicher Länge in der Reinigungsschicht zum Einsatz kommen.

Mit erfindungsgemäßen Wundreinigungseinrichtungen kann nicht nur die Wunde selbst, sondern auch die die Wunde umgebende Haut gereinigt werden. Dabei kann nicht nur Humanmaterial entfernt werden. Zusätzlich oder alternativ zur Entfernung von Humanmaterial kann mit erfindungsgemäßen Wundreinigungseinrichtungen auch Fremdmaterial aus dem Bereich der Wunde und/oder der die Wunde umgebenden Haut entfernt werden. Als Beispiele für Fremdmaterial können Rückstände von Salben, wie etwa Zinksalbe, Rückstände von Pflastern oder Wundabdeckungen, durch Zugabe von Wasser und/oder wässrigen oder nicht-wässrigen Lösungen aus Humanmaterial gebildete Substanzen und dergleichen genannt werden.

Bei erfindungsgemäßen Wundreinigungseinrichtungen können die Abrasionsbereiche eine die Absorptionsbereiche voneinander trennende Linien-, Raster- und/oder Netzstruktur bilden. Dabei beträgt der Abstand zwischen zwei durch einen Abrasionsbereich voneinander getrennten Absorptionsbereichen 10 % oder mehr, vorzugsweise 30 % oder mehr, insbesondere 50 % oder mehr und/oder 150 % oder weniger, insbesondere 100 % oder weniger, besonders bevorzugt 90 % oder weniger der wirksamen Faserlänge der Fasern des Absorptionsbereichs. Die Absorptionsbereiche können ebenso wie die Abrasionsbereiche zusammenhängen. Sie können beispielsweise spiralförmig umlaufen. Der Abstand zwischen zwei durch eine Abrasionsbereich voneinander getrennten Absorptionsbereichen kann 30 mm oder weniger, insbesondere 10 mm oder weniger, ggf. 5 mm oder weniger und/oder 0,1 mm oder mehr, vorzugsweise 1 mm oder mehr, insbesondere 3 mm oder mehr betragen.

Wie vorstehend bereits erläutert, sind Wundreinigungseinrichtungen mit Wundreinigungsschichten aus Fasern mit einer geringen Faserlänge besonders gut geeignet zum Entfernen von viskosen Flüssigkeiten aus Wunden.

Im Rahmen der Erfindung ist auch an die Bereitstellung eines Wundreinigungskits mit einer steril in einer Packung aufgenommenen erfindungsgemäßen Wundreinigungseinrichtung gedacht. Dabei kann in und/oder an der Packung eine Anweisung zur Verwendung der Wundreinigungseinrichtung bei der Behandlung von Brandwunden, und/oder Nekrosen und/oder hartnäckigen Fibrinbelägen und/oder exsudierenden Wunden und/oder fibrinösen Belägen vorgesehen sein.

Wie der vorstehenden Erläuterung erfindungsgemäßer Wundreinigungseinrichtungen und Wundreinigungskits zu entnehmen ist, wird eine erfindungsgemäße Wundreinigungseinrichtung mit besonderem Vorteil zur Herstellung einer Therapieanordnung für die Behandlung von Brandwunden, Nekrosen und/oder Fibrinbelägen verwendet. Die erfindungsgemäße Wundreinigungseinrichtung ist zur Verwendung bei der Behandlung von Brandwunden, Nekrosen und/oder Fibrinbelägen und/oder exsudierenden Wunden geeignet.

Die Abrasionsbereiche erfindungsgemäßer Wundreinigungseinrichtungen können ein quadratisches Muster bilden. Dabei wird der Abstand zwischen benachbarten Absorptionsbereichen als Kantenlänge der Quadrate definiert. Sie können auch spiralförmig umlaufen. Sie können wellenartige Linien bilden. Es ist auch an strahlenartig von einem gemeinsamen Zentrum ausgehende Abrasionsbereiche gedacht. Auch die Ausführung der Abrasionsbereiche in Form von zackenförmigen Linien zwischen einzelnen Absorptionsbereichen ist möglich. Sofern die Abrasionsbereiche durch Nahtlinien gebildet werden, kann der Abstand zwischen den einzelnen Nahtlinien zwischen 20 und 90 % der wirksamen Faserlänge in den Absorptionsbereichen liegen. Bei einer bevorzugten Ausführungsform kann der Abstand zwischen den Nahtlinien etwa 0,5 bis 3 cm, insbesondere etwa 0,7 cm betragen. Bei einer anderen Ausführungsform der Erfindung kann der Abstand zwischen den einzelnen Nahtlinien etwa 1 cm betragen. Bei der Behandlung großflächiger Wunden mit entsprechend dimensionierten Wundreinigungsschichten kann der Abstand zwischen den Nahtlinien 2 bis 3 cm betragen.

Wenn die erfindungsgemäße Wundreinigungseinrichtung nur eine Reinigungsschicht mit einem Reinigungsbereich aus Fasern mit einer Faserlänge von 5 mm oder weniger und 1,5 mm oder mehr aufweist, können damit viskose Flüssigkeiten besonders gut aus der Wunde abgelöst und festgehalten werden. Wenn sowohl Fasern mit einer geringen wirksamen Länge als auch Fasern mit einer vergleichsweise großen wirksamen Länge eingesetzt werden, hat es sich als günstig erwiesen, wenn der Anteil der Fasern mit geringer Länge zwischen 10 und 90 %, vorzugsweise zwischen 30 und 70 %, besonders bevorzugt etwa 50 %, an der Gesamtfaseranzahl beträgt. Die die Reinigungsschicht erfindungsgemäßer Wundreinigungseinrichtungen bildenden Fasern können zumindest teilweise, vorzugsweise vollständig, aus Polyester, Nylon, Vinyl, Polyethylen, Polypropylen, Aramid, Cellulose und/oder Polyamid bestehen. Auf die Oberfläche der einzelnen Fasern können auch Schleifmittel als zusätzliches Reinigungsmittel aufgebracht werden.

Gemäß einem weiteren Gesichtspunkt der Erfindung können zumindest einige Reinigungselemente bzw. Wundreinigungselemente zumindest teilweise mit einer antimikrobiellen Beschichtung ausgestattet sein.

Bei Einsatz der bekannten Wundreinigungseinrichtungen ist auch daran gedacht, bakterielle Besiedelungen der Wunde mit einem Biofilm, welcher zur systematischen Infektion beim Patienten führt, zu beseitigen. Zu diesem Zweck können die aus Kunststofffasern ausgeführten Wundreinigungselemente bei den bekannten Wundreinigungseinrichtungen auch mit einer antimikrobiell wirksamen Beschichtung ausgestattet sein.

Bei Einsatz der bekannten Wundreinigungseinrichtungen hat es sich jedoch gezeigt, dass es in vielen Fällen trotz sorgfältiger Wundreinigung und Einsatz einer antimikrobiell wirksamen Beschichtung noch zu Infektionen der Wunde kommt.

Im Rahmen dieser Erfindung werden diese Probleme durch eine Weiterbildung der bekannten Wundreinigungseinrichtungen gelöst, die im Wesentlichen dadurch gekennzeichnet ist, dass die antimikrobielle Beschichtung zwei sich voneinander unterscheidende, vorzugsweise als Bi-Metallpartikel vorliegende Metalle aufweist.

Die Erfindung geht auf die Erkenntnis zurück, dass herkömmliche Beschichtungen ihre antimikrobielle Wirkung bei der Wundreinigung kaum entfalten. Bei der Wundreinigung mit erfindungsgemäßen Wundreinigungseinrichtungen wird die Wunde unter Einsatz der Wundreinigungselemente ausgewischt. Die Kontaktzeit zwischen Wundreinigungselementen und Wundbett bzw. bakterieller Besiedelung der Wunde bzw. die Wirkdauer beträgt dabei allenfalls einige Sekunden. Andererseits beruht der Wirkmechanismus herkömmlicher antimikrobieller Beschichtungen, beispielsweise auf Silberbasis, darauf, dass Metall- bzw. Silberionen freigesetzt werden und ihre oligodynamische Wirkung entfalten. Die Freisetzung der Metall- bzw. Silberionen erfolgt allerdings mit starker Verzögerung, so dass die antimikrobielle Wirkung bei einer Kontaktzeit von nur einigen Sekunden nicht oder kaum einsetzt.

Andererseits weisen Beschichtungen mit antimikrobiell wirksamen Substanzen in Form von großen Molekülen, wie etwa PHMB, nur eine reduzierte Eindringtiefe in das Wundbett auf, was für die angestrebte antimikrobielle Wirkung auch nicht ausreicht.

Dieser Gesichtspunkt der Erfindung beruht auf der überraschenden Erkenntnis, dass die antimikrobielle Wirkung bei Einsatz von Beschichtungen mit zwei sich voneinander unterscheidenden Metallen, die vorzugsweise als Bi-Metallpartikel vorliegen, anders als in der EP 2 077 976 B1 angenommen, nicht auf der Freisetzung von Metallionen beruht, sondern auf einer katalytischen Wirkung, mit deren Hilfe bei Kontakt mit wässrigen Medien antimikrobiell wirksame Substanzen, insbesondere reaktive Sauerstoffspezies (ROS = Reactive Oxygene Species), wie etwa Wasserstoffperoxid, erzeugt werden. Die katalytische Erzeugung der antimikrobiell wirksamen Substanzen unter Einsatz einer zwei Metallspezies enthaltenden Beschichtung erfolgt dabei bei Kontakt mit wässrigen Medien auf einer Zeitskala von deutlich weniger als einer Sekunde. Daher kommt es beim Auswischen einer Wunde mit gemäß diesem Gesichtspunkt ausgestalteten Wundreinigungseinrichtungen bei entsprechenden Kontaktzeiten zwischen Wundreinigungselementen und Wundbett zu einer ausreichenden katalytischen Umsetzung zu antimikrobiell wirksamen Produkten. Ferner handelt es sich bei den so erzeugten antimikrobiell wirksamen Produkten in Form von ROS um vergleichsweise kleine Moleküle, die eine ausreichende Eindringtiefe in das Wundbett aufweisen. Insgesamt kann so einer bakteriellen Besiedelung des Wundbetts unter Einsatz erfindungsgemäßer Wundreinigungseinrichtungen zufriedenstellend begegnet werden.

Sofern die Metallspezies in Form von Bi-Metallpartikeln vorliegen, kommt es zur Bildung eines Kontaktpotenzials, mit dem die katalytische Wirkung der Metallspezies weiter gefördert werden kann. Ein weiterer Vorteil der erfindungsgemäß eingesetzten antimikrobiellen Beschichtung ist darin zu sehen, dass sich die Beschichtung nicht verbraucht, weil die antimikrobielle Wirkung nicht auf der Freisetzung von Metallionen beruht, sondern auf einer katalytischen Wirkung. Daher kann es zu einer fortlaufenden Neubildung relativ kurzlebiger reaktiver Substanzen, insbesondere ROS kommen. Die antimikrobielle Wirkung bleibt daher auch bei wiederholtem Einsatz, wie etwa bei mehrmaligem Auswischen derselben Wunde mit ein und demselben Wundreinigungselement, erhalten.

Im Rahmen der Erfindung hat es sich als besonders günstig erwiesen, wenn die antimikrobielle Beschichtung Silber und/oder Ruthenium enthält. Die antimikrobielle Wirkung von Silber-Ruthenium-Beschichtungen ist beispielsweise in der EP 2 077 976 B1 beschrieben. Der dort angenommene Wirkmechanismus (Abgabe von Silberionen) spielt bei der Anwendung entsprechender Beschichtungen im Zusammenhang mit Wundreinigungseinrichtungen keine Rolle. Überraschenderweise hat es sich gezeigt, dass Silber-Ruthenium-Beschichtungen die katalytische Umsetzung wässriger Medien zu reaktiven Sauerstoffspezies, die antimikrobiell wirksam sein können, begünstigt. Erst dadurch ist der Einsatz dieser Beschichtung im Zusammenhang mit der Wundreinigung durch Auswischen von Wunden sinnvoll geworden.

Erstaunlicherweise wird die katalytische Wirkung begünstigt, wenn die antimikrobielle Beschichtung ein Vitamin und/oder ein Vitaminderivat aufweist, wobei das Vitamin vorzugsweise Ascorbinsäure ist.

Die Dicke der antimikrobiell wirksamen Beschichtung auf den Reinigungselementen beträgt vorzugsweise weniger als 1 µm, insbesondere 800 nm oder weniger. Dadurch wird gewährleistet, dass auch bei Einsatz feiner Fasern keine bedeutende Änderung der mechanischen Fasereigenschaften durch die Beschichtung auftritt. Zur Gewährleistung der Wirksamkeit der Beschichtung hat es sich als zweckmäßig erwiesen, wenn die Dicke der Beschichtung 100 nm oder mehr, insbesondere 200 nm oder mehr aufweist. Die Beschichtung wird zweckmäßigerweise mittels PVD (physical vapor disposition)-Techniken auf die Wundreinigungselemente aufgebracht.

Zusätzlich oder alternativ kann die antimikrobielle Beschichtung auch eine oberflächenaktive Substanz aufweisen. Ebenso wie bei den in der WO 2010/085831 A1 beschriebenen Wundreinigungseinrichtungen können die Wundreinigungselemente einer erfindungsgemäßen Wundreinigungseinrichtung von einer Trägerschicht abstehen, wobei zumindest einige der Wundreinigungselemente vorzugsweise auf ihrer von der Trägerschicht abgewandten Seite frei auskragende Enden aufweisen können.

Wie der vorstehenden Erläuterung erfindungsgemäßer Wundreinigungseinrichtungen zu entnehmen ist, werden diese mit besonderem Vorteil beim Debridement verwendet. Dabei versteht man unter dem Ausdruck Debridement die Wundbettpräparation, bei der vom Körper selbst gebildete Substanzen bzw. Humanmaterial, wie zum Beispiel überschießende Flüssigkeit, Fibrinbeläge, abgestorbenes Gewebe der Oberhaut, wie zum Beispiel überschießendes Hornmaterial, oder tote Hornzellen und/oder Beläge aus abgestorbenem Gewebe (Nekrosen) entfernt werden.

Die Erfindung ist nicht auf den Einsatz von Silber-Ruthenium-haltigen Beschichtungen beschränkt. Vielmehr ist auch an den Einsatz von Beschichtungen mit Platin-Ruthenium-, Ruthenium-Kupfer- und/oder Ruthenium-Gold-Nanopartikeln gedacht.

Im Rahmen der Erfindung kann die erfindungsgemäße Wundreinigungseinrichtung in Form eines Kits bereitgestellt werden, bei dem die Wundreinigungseinrichtung steril in einer Packung aufgenommen ist. In und/oder an der Packung kann eine Anweisung zur Verwendung der Wundreinigungseinrichtung bei der Behandlung von Brandwunden, Nekrosen und/oder hartnäckigen Fibrinbelägen und/oder Biofilmen und/oder einer bakteriellen Belastung vorgesehen sein.

Wie der vorstehenden Erläuterung erfindungsgemäßer Wundreinigungseinrichtungen und -kits zu entnehmen ist, bezieht sich die Erfindung auch auf die Verwendung einer erfindungsgemäßen Wundreinigungseinrichtung zur Herstellung einer Therapieanordnung für die Behandlung von Brandwunden, Nekrosen und/oder Fibrinbelägen. Zu diesem Zweck kann die erfindungsgemäße Wundreinigungseinrichtung auch an einem vorzugsweise biegsamen Applikationsstab angebracht werden. Der Applikationsstab kann ggf. auch beidseitig mit einer erfindungsgemäßen Wundreinigungseinrichtung ausgestattet werden. Bei anderen Ausführungsformen der Erfindung kann die Wundreinigungseinrichtung auch als modulares System mit einem ggf. lösbar an einem Applikationsstab befestigbaren Reinigungskopf ausgeführt sein, wobei der Reinigungskopf eine erfindungsgemäße Wundreinigungseinrichtung aufweist. Ferner kann eine erfindungsgemäße Wundreinigungseinrichtung auch in Form eines Handschuhs ausgeführt sein.

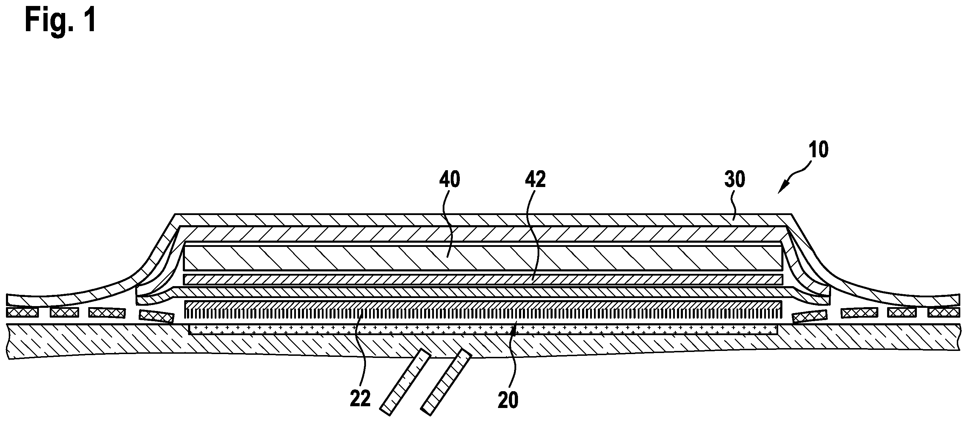

## Patentansprüche

1. Wundreinigungseinrichtung mit einer zum Ablösen von Substanzen aus einer Wunde und zum Festhalten der Substanzen ausgelegten Wundreinigungsschicht mit einer Anzahl von strangförmigen Reinigungselementen, wobei der Reinigungsquotient R = (E*F)/l mindestens einiger Reinigungselemente 1 N/mm oder größer, und 100 N/mm oder kleiner ist, wobei E den Elastizitätsmodul des Materials bezeichnet, aus dem die Reinigungselemente bestehen, F die mittlere Querschnittsfläche der Reinigungselemente in einer senkrecht zur Strangachse verlaufenden Richtung und 1 die wirksame Länge der Reinigungselemente bezeichnet, wobei die Reinigungselemente Chemiefasern, insbesondere Monofilamente und/oder Multifilamente aus Kunststoff aufweisen, **dadurch gekennzeichnet, dass** die Reinigungselemente von einer Trägerschicht ausgehende Schlaufen bilden, wobei die wirksame Länge als die Hälfte der Länge der Schlaufe definiert ist, gemessen zwischen den beiden Punkten, an denen sie aus der Trägerschicht austritt und die Wundreinigungsschicht zwei, drei oder mehr streifenförmig nebeneinander liegende Wundreinigungsbereiche mit sich voneinander unterscheidenden Wundreinigungseigenschaften aufweist, wobei einer der Wundreinigungsbereiche ein zum Absorbieren von Wundflüssigkeiten ausgelegter Absorptionsbereich ist, einer der Wundreinigungsbereiche ein zum Ablösen von Fibrinbelägen, abgestorbenem Gewebe, Hornmaterial oder dergleichen ausgelegter Abrasionsbereich ist, wobei zumindest einige Reinigungselemente als ggf. verdrillte Bündel von zwei, drei oder mehr Monofilamenten ausgeführt sind,
die Chemiefasern einen Durchmesser von 500 µm oder weniger, insbesondere 150 µm oder weniger aufweisen,
die Chemiefasern einen Durchmesser von 5 µm oder mehr aufweisen,
die wirksame Länge der Schlaufen bevorzugt 3 mm oder mehr beträgt,
die wirksame Länge der Schlaufen 11 mm oder weniger beträgt, und
der Elastizitätsmodul des Materials der Chemiefasern 50.000 N/mm² oder weniger, 500 N/mm² oder mehr, beträgt und
mindestens einige Reinigungselemente zumindest teilweise, vorzugsweise vollständig aus Polyester bestehen.

2. Wundreinigungseinrichtung nach Anspruch 1, **dadurch gekennzeichnet, dass** auf der Oberfläche mindestens eines Reinigungselements ein Schleifmittel aufgebracht ist.

3. Wundreinigungseinrichtung nach Anspruch 2, **dadurch gekennzeichnet, dass** das Schleifmittel Korund, Zirkon, Siliziumcarbid, Bornitrid, Chromoxid, Flint, Schmirgel, Granat, Bornitrid, insbesondere kubisches Bornitrid und/oder Diamant aufweist.

4. Wundreinigungseinrichtung nach Anspruch 3, **dadurch gekennzeichnet, dass** das Schleifmittel eine Körnung im Bereich zwischen 50 und 1000 Mesh, insbesondere zwischen 150 und 800 Mesh aufweist.

5. Wundreinigungseinrichtung nach einem der vorhergehenden Ansprüche, bei der zumindest einige Reinigungselemente zumindest teilweise mit einer antimikrobiellen Beschichtung ausgestattet sind, **dadurch gekennzeichnet, dass** die antimikrobielle Beschichtung zwei sich voneinander unterscheidende, vorzugsweise als Bi-Metallpartikel vorliegende Metalle aufweist.

6. Wundreinigungseinrichtung nach Anspruch 5, **dadurch gekennzeichnet, dass** die antimikrobielle Beschichtung Silber und/oder Ruthenium aufweist.

7. Wundreinigungseinrichtung nach Anspruch 5 oder 6, **dadurch gekennzeichnet, dass** die antimikrobielle Beschichtung ein Vitamin oder ein Vitaminderivat aufweist, wobei das Vitamin vorzugsweise Ascorbinsäure ist.

8. Wundreinigungseinrichtung nach einem der Ansprüche 5 bis 7, **dadurch gekennzeichnet, dass** die antimikrobielle Beschichtung eine oberflächenaktive Substanz aufweist.

9. Wundreinigungskit mit einer steril in einer Packung aufgenommenen Wundreinigungseinrichtung nach einem der vorhergehenden Ansprüche.

10. Wundreinigungskit nach Anspruch 9, **dadurch gekennzeichnet, dass** in und/oder an der Packung eine Anweisung zur Verwendung der Wundreinigungseinrichtung bei der Behandlung von Brandwunden, Nekrosen und/oder hartnäckigen Fibrinbelägen und/oder exsudierenden Wunden und/oder Biofilmen und/oder einer bakteriellen Belastung vorgesehen ist.

## Claims

1. A wound cleaning device with a wound cleaning layer designed for detaching substances from a wound and for holding the substances in place, with a number of strand-shaped cleaning elements, wherein the cleaning quotient R = (E*F)/l of at least some cleaning elements is 1 N/mm or greater, and 100 N/mm or less, wherein E denotes the modulus of elasticity of the material of which the cleaning elements consist, F denotes the average cross-sectional area of the cleaning elements in a direction running perpendicularly to the strand axis, and l denotes the effective length of the cleaning elements, wherein the cleaning elements comprise chemical fibres, in particular monofilaments and/or multifilaments of plastic, **characterized in that** the cleaning elements form loops starting from a carrier layer, wherein the effective length is defined as half the length of the loop, measured between the two points at which it emerges from the carrier layer, and the wound cleaning layer comprises two, three or more wound cleaning regions lying next to one another in the form of strips with wound cleaning properties differing from one another, wherein one of the wound cleaning regions is an absorption region designed for absorbing wound fluids, one of the wound cleaning regions is an abrasion region designed for detaching fibrin coatings, dead tissue, horn material or the like, wherein at least some cleaning elements are designed as optionally twisted bundles of two, three or more monofilaments,
the chemical fibres have a diameter of 500 µm or less, in particular 150 µm or less,
the chemical fibres have a diameter of 5 µm or more,
the effective length of the loops is preferably 3 mm or more,
the effective length of the loops is 11 mm or less, and
the modulus of elasticity of the material of the chemical fibres is 50,000 N/mm2 or less, 500 N/mm2 or more, and
at least some cleaning elements consist at least partially, preferably completely, of polyester.

2. The wound cleaning device according to claim 1, **characterized in that** an abrasive is applied to the surface of at least one cleaning element.

3. The wound cleaning device according to claim 2, **characterized in that** the abrasive comprises corundum, zirconium, silicon carbide, boron nitride, chromium oxide, flint, emir, garnet, boron nitride, in particular cubic boron nitride and/or diamond.

4. The wound cleaning device according to claim 3, **characterized in that** the abrasive has a grain size in the range between 50 and 1000 mesh, in particular between 150 and 800 mesh.

5. The wound cleaning device according to one of the preceding claims, in which at least some cleaning elements are at least partially equipped with an antimicrobial coating, **characterized in that** the antimicrobial coating comprises two metals which differ from one another and are preferably present as bi-metal particles.

6. The wound cleaning device according to claim 5, **characterized in that** the antimicrobial coating comprises silver and/or ruthenium.

7. The wound cleaning device according to claim 5 or 6, **characterized in that** the antimicrobial coating comprises a vitamin or a vitamin derivative, wherein the vitamin is preferably ascorbic acid.

8. The wound cleaning device according to one of claims 5 to 7, **characterized in that** the antimicrobial coating comprises a surface-active substance.

9. A wound cleaning kit comprising a wound cleaning device according to one of the preceding claims accommodated in a sterile manner in a package.

10. The wound cleaning kit according to claim 9, **characterized in that** instructions for using the wound cleaning device in the treatment of burnt wounds, necroses and/or stubborn fibrin coatings and/or exuding wounds and/or biofilms and/or a bacterial load are provided in and/or on the package.

## Revendications

1. Dispositif de nettoyage de plaies avec une couche de nettoyage de plaies conçue pour détacher des substances d'une plaie et pour retenir les substances, avec un certain nombre d'éléments de nettoyage en forme de cordons, le quotient de nettoyage R = (E*F)/l d'au moins certains éléments de nettoyage étant de 1 N/mm ou plus, et 100 N/mm ou inférieur, E désignant le module d'élasticité du matériau dont sont constitués les éléments de nettoyage, F désignant la section transversale moyenne des éléments de nettoyage dans une direction perpendiculaire à l'axe du cordon et l désignant la longueur effective des éléments de nettoyage, les éléments de nettoyage comportant des fibres chimiques, en particulier des monofilaments et/ou des multifilaments en matière plastique, **caractérisé en ce que** les éléments nettoyants forment des boucles partant d'une couche support, la longueur effective étant définie comme la moitié de la longueur de la boucle, mesurée entre les deux points où elle sort de la couche support, et la couche de nettoyage des plaies présente deux, trois ou plusieurs zones de nettoyage de plaies disposées en bandes juxtaposées et présentant des propriétés de nettoyage de plaies différentes les unes des autres, l'une des zones de nettoyage de plaies étant une zone d'absorption conçue pour absorber les liquides de la plaie, l'une des zones de nettoyage de plaies étant une zone de nettoyage de plaies conçue pour détacher les dépôts de fibrine, les tissus nécrosés, la de la kératine ou similaire, au moins certains éléments nettoyants étant réalisés sous forme de faisceaux éventuellement torsadés de deux, trois ou plusieurs monofilaments,
les fibres chimiques ont un diamètre de 500 µm ou moins, en particulier de 150 µm ou moins,
les fibres chimiques ont un diamètre de 5 µm ou plus,
la longueur effective des boucles est de préférence de 3 mm ou plus,
la longueur effective des boucles est inférieure ou égale à 11 mm, et
le module d'élasticité du matériau des fibres chimiques est inférieur ou égal à 50 000 N/mm², supérieur ou égal à 500 N/mm², et
au moins certains éléments de nettoyage sont constitués au moins en partie, de préférence entièrement, de polyester.

2. Dispositif de nettoyage de plaies selon la revendication 1, **caractérisé en ce qu'**un abrasif est appliqué sur la surface d'au moins un élément de nettoyage.

3. Dispositif de nettoyage de plaies selon la revendication 2, **caractérisé en ce que** l'abrasif comprend du corindon, du zircon, du carbure de silicium, du nitrure de bore, de l'oxyde de chrome, du silex, de l'émeri, du grenat, du nitrure de bore, en particulier du nitrure de bore cubique et/ou du diamant.

4. Dispositif de nettoyage de plaies selon la revendication 3, **caractérisé en ce que** l'abrasif présente une granulométrie comprise entre 50 et 1000 mesh, en particulier entre 150 et 800 mesh.

5. Dispositif de nettoyage de plaies selon l'une des revendications précédentes, dans lequel au moins certains éléments de nettoyage sont au moins partiellement pourvus d'un revêtement antimicrobien, **caractérisé en ce que** le revêtement antimicrobien comprend deux métaux différents, de préférence sous forme de particules bimétalliques.

6. Dispositif de nettoyage de plaies selon la revendication 5, **caractérisé en ce que** le revêtement antimicrobien comprend de l'argent et/ou du ruthénium.

7. Dispositif de nettoyage de plaies selon la revendication 5 ou 6, **caractérisé en ce que** le revêtement antimicrobien comprend une vitamine ou un dérivé de vitamine, la vitamine étant de préférence l'acide ascorbique.

8. Dispositif de nettoyage de plaies selon l'une des revendications 5 à 7, **caractérisé en ce que** le revêtement antimicrobien contient une substance tensioactive.

9. Kit de nettoyage de plaies comprenant un dispositif de nettoyage de plaies stérilisé dans un emballage selon l'une des revendications précédentes.

10. Kit de nettoyage de plaies selon la revendication 9, **caractérisé en ce que** des instructions d'utilisation du dispositif de nettoyage de plaies pour le traitement des brûlures, des nécroses et/ou des dépôts de fibrine tenaces et/ou des plaies exsudatives et/ou des biofilms et/ou d'une charge bactérienne sont prévues dans et/ou sur l'emballage.
